# FASCICULE DE BREVET EUROPEEN

(11) **EP 0 938 891 B1**
(45) Date de publication et mention de la délivrance du brevet: **05.01.2005**
(21) Numéro de dépôt: 98402959.5
(22) Date de dépôt: 26.11.1998
(51) Int. Cl.: A61K 7/48

(54) **Composition cosmétique contenant de l'acide cinnamique et son utilisation**
Zimtsäure enthaltende kosmetische Zusammensetzung und ihre Verwendung
Cosmetic composition containing cinnamic acid and its use

(30) Priorité: 19.12.1997 FR 9716180
(43) Date de publication de la demande: 01.09.1999
(73) Titulaire: L'OREAL, 75008 Paris (FR)
(72) Inventeur: Breton, Lionel, 78000 Versailles (FR); Girerd-Dugue, Florence, 34130 Maugio (FR); Renault, Béatrice, 94410 Saint Maurice (FR)
(74) Mandataire: Tezier Herman, Béatrice

(56) Documents cités:
- EP-A- 0 664 290
- EP-A- 0 716 847
- US-A- 5 093 109
- DATABASE WPI Week 8802 Derwent Publications Ltd., London, GB; AN 88-012502 XP002081837 "Hair rinsing composition contains hops, nettle and chestnut extract, safflower oil cake protein production byproduct and isopropyl palmitate" A & SU 1 311 734 A (BIOKHIMREAKTIV COMB) 23 mai 1987
- PATENT ABSTRACTS OF JAPAN vol. 017, no. 475 (C-1103), 30 août 1993 & JP 05 117145 A (KANEBO LTD), 14 mai 1993
- PATENT ABSTRACTS OF JAPAN vol. 017, no. 674 (C-1140), 10 décembre 1993 & JP 05 221845 A (HISAMITSU PHARMACEUT CO), 31 août 1993
- PATENT ABSTRACTS OF JAPAN vol. 017, no. 055 (C-1023), 3 février 1993 & JP 04 266807 A (SOUKEN KK), 22 septembre 1992
- PATENT ABSTRACTS OF JAPAN vol. 018, no. 118 (C-1172), 25 février 1994 & JP 05 310526 A (EISAI CO LTD), 22 novembre 1993
- DATABASE WPI Week 8936 Derwent Publications Ltd., London, GB; AN 89-258961 XP002081819 & JP 01 186811 A (SUNSTAR KK) 26 juillet 1989
- DATABASE WPI Week 9407 Derwent Publications Ltd., London, GB; AN 94-053956 XP002081878 & JP 06 009603 A (HASEGAWA CO LTD) 18 janvier 1994
- W.A. POUCHER: "Poucher's Perfumes, Cosmetics and Soaps. Volume 1" , CHAPMAN & HALL , LONDON XP002081818 * page 84-89 *
- P. ROVESTI: "Recherches sur l'action des auxines et des phytohormones en cosmétique" PARFUMERIE MOD., vol. 48, no. 54, 1956, pages 86-91, XP002082741

## Description

L'invention se rapporte à l'utilisation de l'acide cinnamique dans une composition cosmétique destinée à stimuler la restructuration de la peau et/ou des muqueuses par la stimulation de la synthèse du collagène.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également constitué de vaisseaux sanguins et de fibres nerveuses. Dans une peau normale, c'est à dire non pathologique ni cicatricielle, le fibroblaste est à l'état quiescent, c'est à dire non prolifératif, peu actif d'un point de vue métabolique et non mobile.

Ce sont les fibres de collagène qui assurent la solidité du derme. Elles sont trés résistantes mais sensibles à certaines enzymes appelées collagénases. Dans le derme, les fibres de collagène sont constituées de fibrilles scellées les unes aux autres, formant ainsi plus de dix types de structures différentes. La solidité du derme est en grande partie due à l'enchevêtrement des fibres de collagène tassées les unes contre les autres en tous sens. Les fibres de collagène participent à l'élasticité et à la tonicité de la peau et/ou des muqueuses.
Les fibres de collagènes sont constamment renouvelées mais ce renouvellement diminue avec l'âge ce qui entraîne un amincissement du derme. Mais, cet amincissement du derme est également dû à des causes pathologiques comme par exemple l'hypersécrétion d'hormones corticoïdes, certaines maladies (syndrome de Marfan, syndrome de Ehlers-Danlos) ou encore des carences vitaminiques (scorbut). Il est également admis que des facteurs extrinsèques comme les rayons ultra-violets, le tabac ou certains traitements (Acide rétinoïque et dérivés, glucocorticoïdes, vitamine D et dérivés par exemple) ont également un effet sur la peau et sur son taux de collagène. Une dégradation des fibres de collagène entraîne l'apparence de peau mole et ridée qui est depuis toujours combattue, l'être humain préférant l'apparence d'une peau lisse et tendue.

Par ailleurs, à la ménopause, les principales modifications concernant le derme sont une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des fines rides et ridules de surface. La peau présente un aspect rugueux à la palpation. Enfin la peau présente une souplesse diminuée.
Il est démontré que les femmes perdent 2,1% de leur taux de collagène par an après la ménopause et que 30% sont perdus dans les cinq premières années postménopausiques.

On comprend donc l'importance que revêt la présence de fibres de collagène dans la peau et l'importance qu'il existe à maintenir, voire renforcer, leur présence.

Il est donc important de pouvoir disposer de produits dont les effets visent à maintenir le taux de collagène dans la peau et lui maintenir une apparence lisse et tendue.

A cet égard la demanderesse a de manière surprenante et inattendue découvert que l'acide cinnamique présente la propriété de stimuler la synthèse du collagène.

L'acide cinnamique est présent sous forme trans dans les huiles essentielles de basilic ou de cannelle, dans la balsamine du Pérou ou encore dans les feuilles de cacao. La forme cis est présente dans l'huile *d'Alpinia malacensis*.

Dans l'art antérieur l'acide cinnamique ou ses dérivés sont connus pour être utilisés dans des compositions pour la prévention des escarres (JP 07 242 558), comme actif anti-ultraviolets (US 5 093 109), dans des compositions pour permanente (DE 3 301 515, DE 2 912 427, EP 22 996), dans des lotions capillaires (JP 7 053 401, JP 3 041 413), dans des compositions dépigmentantes (JP 5 221 845, JP 1 186 811), comme anti-oxydant (EP 664 290).

A la connaissance de la demanderesse il n'a jamais été décrit dans l'état de la technique l'utilisation de l'acide cinnamique pour stimuler la synthèse du collagène.

L'invention a donc pour objet l'utilisation dans une composition cosmétique d'une quantité efficace d'acide cinnamique, la composition étant destinée à stimuler la synthèse du collagène.

Ainsi, selon l'invention, l'acide cinnamique peut être d'origine naturelle ou synthétique. Par origine naturelle, on entend l'acide cinnamique préparé à partir de matériel végétal dans lequel ils se trouvent présents à l'état naturel. Par origine synthétique, on entend l'acide cinnamique préparé par synthèse chimique ou par biotechnologie.
Ainsi, par la suite dans le texte le terme acide cinnamique s'entend comme désignant de l'acide cinnamique d'origine naturelle ou synthétique purifié ou toute préparation le contenant.

Bien entendu, il est possible d'utiliser selon l'invention l'acide cinnamique seul ou en mélange.

Particulièrement l'acide cinnamique ou la composition le contenant sont utilisés selon l'invention en application topique sur la peau et/ou les muqueuses.

On a vu précédemment que le collagène est impliqué dans la solidité du derme, donc dans la fermeté de la peau et/ou des muqueuses.

Ainsi, un des aspects de l'invention est donc de proposer l'utilisation dans une composition cosmétique d'une quantité efficace d'acide cinnamique, l'acide cinnamique ou la composition étant destiné à diminuer les signes cutanés du vieillissement, plus particulièrement diminuer l'apparence de la peau molle et/ou ridée.

Un autre aspect de l'invention est donc de proposer l'utilisation dans une composition cosmétique d'une quantité efficace d'acide cinnamique, l'acide cinnamique ou la composition étant destiné à stimuler le raffermissement de la peau et/ou des muqueuses.

Selon aussi un autre aspect, l'invention a pour objet l'utilisation dans une composition cosmétique d'une quantité efficace d'acide cinnamique, l'acide cinnamique ou la composition étant destiné à favoriser le lissage de la peau et/ou pour tendre la peau.

Selon encore un autre aspect, l'invention a pour objet l'utilisation dans une composition cosmétique d'une quantité efficace d'acide cinnamique, l'acide cinnamique ou la composition étant destiné à combattre les effets cutanés de la ménopause, plus particulièrement les effets de la ménopause sur le collagène.

La quantité d'acide cinnamique utilisable selon l'invention dépend bien évidemment de l'effet recherché et doit être en une quantité efficace pour stimuler la synthèse du collagène.

A titre d'exemple la quantité d'acide cinnamique utilisable selon l'invention peut aller par exemple de 10⁻⁶% à 10% et de préférence de 10⁻³% à 5% du poids total de la composition.

Il est possible d'utiliser dans les compositions de l'invention de l'acide cinnamique en association avec un autre produit stimulant la synthèse du collagène.
Parmi ces autres produits stimulant la synthèse du collagène on peut citer les hormones végétales ou encore la vitamine C ou ses dérivés.

Parmi les hormones végétales on peut citer les auxines telles que l'acide 3-indolacétique (IAA), l'acide 4-chloroindole-3-acétique (4-Cl-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'idole acétaldéhyde et l'indole acétonitrile.

Préférentiellement, selon l'invention on utilise l'acide β-naphtoxyacétique.

La peau étant constituée de bien d'autres composants que le collagène, il s'avère intéressant lorsque l'on favorise sa synthèse par de l'acide cinnamique de favoriser en même temps la synthèse de ces autres composants comme par exemple les lipides.

A cet égard les hormones végétales comme les auxines et particulièrement l'acide β-naphtoacétique peuvent encore être citées.

L'invention a pour objet une composition cosmétique comprenant dans un milieu cosmétiquement acceptable une quantité efficace d'acide cinnamique et au moins un composé choisi parmi un composé stimulant la synthèse des lipides et un autre composé stimulant la synthèse du collagène.

Par milieu cosmétiquement acceptable on entend un milieu compatible avec la peau, les muqueuses. les ongles. les cheveux.

La composition selon l'invention comprend bien évidemment un support cosmétiquement acceptable et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse. dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Il est également possible d'utiliser selon l'invention en association avec l'acide cinnamique, des composés choisis parmi
- les hormones végétales ;
- les agents antibactériens tels que les macrolides, les pyranosides et les tétracyclines, et notamment l'érythromycine ;
- les agents antagonistes de calcium, comme le vérapamil et le diltiazem ;
- des capteurs de radicaux OH, tels que le diméthylsulfoxyde ;
- des extraits de végétaux tels que ceux d'lridacés ou de soja, extraits pouvant alors contenir ou non des isoflavones ;
- des extraits de micro-organismes dont en particulier des extraits bactériens comme ceux de bactéries filamenteuses non photosynthétiques.

A la liste ci-dessus, d'autres composés peuvent également être ajoutés, à savoir par exemple les ouvreurs de canaux potassiques tels que le diazoxyde et le minoxidil, la spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés.

Selon l'invention, on peut, entre autres, associer l'acide cinnamique à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les agents modulant l'adhésion bactérienne sur la peau et /ou les muqueuses tels que le miel, notamment le mile d'acacias et certains dérivés de sucres ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes. tels que l'amphotéricine B, les composés de la famille des allylamines, tels que la terbinafine, ou encore l'octopirox ;
- les agents antiviraux tels que l'acyclovir;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bèta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.
- des substances telles que les antagonistes de substance P, de CGRP ou de bradykinine ou les inhibiteurs de NO synthase, composés décrits comme étant actifs dans le traitement des peaux sensibles et comme présentant des effets anti-irritants, en particulier vis-à-vis de composés irritants éventuellement présents dans les compositions.

Ainsi, un autre objet de l'invention concerne une composition comprenant une quantité efficace de l'acide cinnamique et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques, les agents modulant la différenciation et/ou la prolifération et/ou la pigmentation cutanée, les antagonistes de substance P, de CGRP ou de bradykinine ou les inhibiteurs de NO synthase.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu), et leurs mélanges.

On peut aussi ajouter des actifs antirides, et notamment des produits tenseurs tels que les protéines végétales et leurs hydrolysats, en particulier l'extrait de protéines de soja vendue sous le nom d'Eleseryl® par la société LSN ou le dérivé d'avoine vendu sous la dénomination Reductine® par la société Silab.

Bien entendu, l'acide cinnamique peut être utilisé dans la préparation de compositions cosmétiques et/ou pharmaceutiques, particulièrement dermatologiques, destinées à stimuler la synthèse du collagène.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

### Exemple 1: Etude de l'effet de l'acide cinnamique sur la synthèse du collagène.

L'étude est faite par mesure de l'incorporation de proline radioactive dans des cultures de fibroblastes dermiques humains normaux.
Les cultures de fibroblastes sont effectuées selon les méthodes classiques de culture cellulaire, à savoir en milieu MEM/M199 vendu par la société Gibco, en présence de bicarbonate de sodium (1,87 mg/ml) de L-glutamine (2 mM), de pénicilline (50 Ul/ml) et de 10% de sérum de veau foetal (Gibco).

Le test est effectué sur des cultures de cellules à 80% de confluence en plaque de 24 trous. L'acide cinnamique, à la concentration de 10⁻⁴M, est mis en contact avec les cellules pendant 48 heures. Le marquage à la proline tritiée (L-[2,3-³H]-proline vendue par Amersham, 33 µCi/ml) est effectué pendant 24 heures. Le taux de proline tritiée incorporée est mesuré en fin de test par précipitation acide des protéines sur filtres et comptage en scintillation liquide.

Les résultats sont évalués par rapport à un témoin constitué par des cellules n'ayant pas été traitées à l'acide cinnamique.

Un témoin positif (vitamine C à 20µg/ml) connue pour stimuler la synthèse du collagène et un témoin négatif (l'acide rétinoïque à 10⁻⁶M) connu pour inhiber la synthèse du collagène sont introduits dans le test comme référence.

Les résultats de ce test sont présentés dans le tableau suivant.

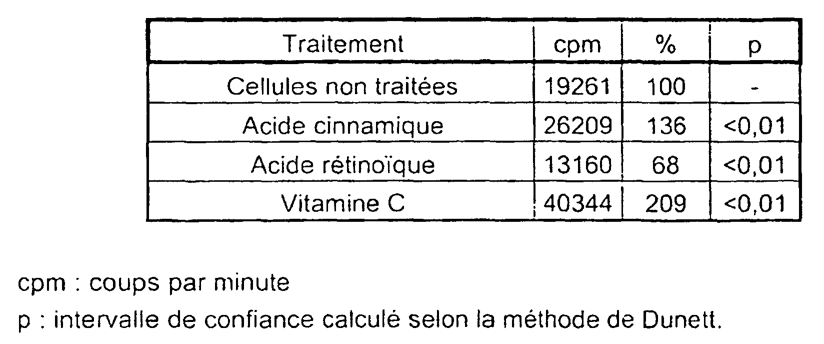

Ces résultats montrent que l'acide cinnamique stimule de façon significative l'incorporation de proline dans le collagène et donc qu'il présente un effet sur la néosynthèse du collagène.

### Exemple 2 : Exemples de compositions selon l'invention. Ces compositions sont obtenues par les techniques habituelles couramment utilisées en cosmétique ou en pharmacie.

| Composition 1 : crème de soin | |
|---|---|
| Cire d'abeille | 1,5 % |
| Huile d'amandes d'abricot | 13,0 % |
| Conservateurs | 0,3 % |
| Parfum | 0,4 % |
| Acide β-naphtoxyacétique | 0,01 % |
| Acide cinnamique | 0,01 % |
| Xanthane | 0,5 % |
| Cyclopenta diméthylsiloxane | 5,0 % |
| Eau déminéralisée stérilisée | 69,28 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

| Composition 2 : huile corporelle | |
|---|---|
| Huile de vaseline | 47,98 % |
| Huile d'amandes d'abricot | 6,0 % |
| Parfum | 1,0 % |
| Acide β-naphtoxyacétique | 0,01 % |
| Acide cinnamique | 0,01 % |
| Cyclopenta diméthylsiloxane | 45,0 % |

| Composition 3 : lait démaquillant | |
|---|---|
| Palmitate d'éthyl-2 hexyle | 10,5 % |
| Fraction liquide de beurre de karité | 16,5 % |
| Conservateurs | 0,3 % |
| Parfum | 0,15 % |
| Acide β-naphtoxyacétique | 0,01 % |
| Acide cinnamique | 0,01 % |
| Hydroxyde de sodium | 0,04 % |
| Polymère carboxyvinylique | 0,2 % |
| Eau déminéralisée stérilisée | 69,79 % |
| Mélange de cétylstéarylglucoside et d'alcools cétylique et stéarylique | 2,5 % |

| Composition 4 : crème de soin | |
|---|---|
| Cire d'abeille | 1,5 % |
| Huile d'amandes d'abricot | 13,0 % |
| Conservateurs | 0,3 % |
| Parfum | 0,4 % |
| Acide β-naphtoxyacétique | 0,01 % |
| Acide cinnamique | 0,01 % |
| Cinnamate d'éthyle | 0,01 % |
| Xanthane | 0,5 % |
| Cyclopenta diméthylsiloxane | 5,0 % |
| Eau déminéralisée stérilisée | 69,27 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

## Revendications

1. Utilisation dans une composition cosmétique d'une quantité efficace d'acide cinnamique, comme agent destiné à stimuler la synthèse de collagène.

2. Utilisation selon la revendication 1, **caractérisée par le fait que** l'acide cinnamique est destiné à combattre les effets de la ménopause sur le collagène.

3. Utilisation selon la revendication 1, **caractérisée par le fait que** l'acide cinnamique est destiné à diminuer les signes cutanés du vieillissement.

4. Utilisation selon la revendication 1, **caractérisée par le fait que** l'acide cinnamique est destiné à stimuler le raffermissement de la peau et/ou des muqueuses.

5. Utilisation selon la revendication 1, **caractérisée par le fait que** l'acide cinnamique est destiné à favoriser le lissage de la peau et/ou à tendre la peau.

6. Utilisation selon l'une quelconque des revendications précédentes, **caractérisée par le fait que** la composition est utilisée en application topique sur la peau et/ou les muqueuses.

7. Utilisation selon l'une quelconque des revendications précédentes, **caractérisée par le fait que** l'acide cinnamique est présent en une quantité allant de 10⁻⁶% à 10% du poids total de la composition.

8. Utilisation selon l'une quelconque des revendications précédentes, **caractérisée par le fait que** l'acide cinnamique est présent en une quantité allant de 10⁻³% à 5% du poids total de la composition.

9. Utilisation selon l'une quelconque des revendications précédentes, **caractérisée par le fait que** l'acide cinnamique est associé à un autre produit stimulant la synthèse du collagène.

10. Utilisation selon la revendication précédente, **caractérisée par le fait que** l'autre produit stimulant la synthèse du collagène est choisi parmi les hormones végétales et ou la vitamine C ou ses dérivés.

11. Utilisation selon la revendication précédente, **caractérisée par le fait que** l'hormone végétale est une auxine.

12. Utilisation selon la revendication précédente, **caractérisée par le fait que** l'auxine est choisie parmi l'acide indole-3-acétique (IAA), l'acide 4-chloroindole-3-acétique (4-Cl-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'indole acétaldéhyde et l'indole acétonitrile.

13. Utilisation selon la revendication précédente, **caractérisée par le fait que** l'auxine est de l'acide β-naphtoxyacétique.

14. Utilisation selon l'une quelconque des revendications précédentes, **caractérisée par le fait que** l'acide cinnamique est associé à un autre produit stimulant la synthèse des lipides.

15. Utilisation selon la revendication précédente, **caractérisée par le fait que** le produit stimulant la synthèse des lipides est une hormone végétale telle que définie dans les revendications 11 à 13.

16. Utilisation selon l'une quelconque des revendications 9 à 15, **caractérisée par le fait que** l'autre produit stimulant la synthèse du collagène et/ou des lipides est en une quantité comprise entre 10⁻⁶% à 10%, et de préférence entre 10⁻³% à 5% du poids total de la composition.

17. Composition cosmétique comprenant dans un milieu cosmétiquement acceptable au moins une quantité efficace d'acide cinnamique et au moins un autre produit stimulant la synthèse du collagène.

18. Composition selon la revendication précédente, **caractérisée par le fait que** le produit stimulant la synthèse du collagène est choisi parmi les hormones végétales ou la vitamine C et ses dérivés.

19. Composition cosmétique comprenant dans un milieu cosmétiquement acceptable au moins une quantité efficace d'acide cinnamique et au moins un composé stimulant la synthèse des lipides.

20. Composition selon la revendication précédente, **caractérisée par le fait que** le composé stimulant la synthèse des lipides est une hormone végétale.

## Claims

1. Use, in a cosmetic composition, of an effective amount of cinnamic acid as an agent intended to stimulate collagen synthesis.

2. Use according to Claim 1, **characterized in that** the cinnamic acid is intended to combat the effects of the menopause on collagen.

3. Use according to Claim 1, **characterized in that** the cinnamic acid is intended to reduce the signs of ageing on the skin.

4. Use according to Claim 1, **characterized in that** the cinnamic acid is intended to stimulate the firming of the skin and/or mucous membranes.

5. Use according to Claim 1, **characterized in that** the cinnamic acid is intended to promote smoothing of the skin and/or to tighten the skin.

6. Use according to any one of the preceding claims, **characterized in that** the composition is used in topical application on the skin and/or mucous membranes.

7. Use according to any one of the preceding claims, **characterized in that** the cinnamic acid is present in an amount ranging from 10⁻⁶% to 10% of the total weight of the composition.

8. Use according to any one of the preceding claims, **characterized in that** the cinnamic acid is present in an amount ranging from 10⁻³% to 5% of the total weight of the composition.

9. Use according to any one of the preceding claims, **characterized in that** the cinnamic acid is combined with another product which stimulates collagen synthesis.

10. Use according to the preceding claim, **characterized in that** the other product which stimulates collagen synthesis is selected from plant hormones and/or vitamin C or its derivatives.

11. Use according to the preceding claim, **characterized in that** the plant hormone is an auxin.

12. Use according to the preceding claim, **characterized in that** the auxin is chosen from 3-indoleacetic acid (IAA), 4-chloro-3-indoleacetic acid (4-Cl-IAA), phenylacetic acid (PAA), 3-indolebutyric acid (IBA), 2,4-dichlorophenoxyacetic acid (2,4-D), α-naphthaleneacetic acid (α-NAA), β-naphthoxyacetic acid, indolethanol, indoleacetaldehyde and indoleacetonitrile.

13. Use according to the preceding claim, **characterized in that** the auxin is β-naphthoxyacetic acid.

14. Use according to any one of the preceding claims, **characterized in that** the cinnamic acid is combined with another product which stimulates lipid synthesis.

15. Use according to the preceding claim, **characterized in that** the product which stimulates lipid synthesis is a plant hormone as defined in Claims 11 to 13.

16. Use according to any one of Claims 9 to 15, **characterized in that** the other product which stimulates collagen and/or lipid synthesis is in an amount of between 10⁻⁶% and 10%, and preferably between 10⁻³% and 5%, of the total weight of the composition.

17. Cosmetic composition comprising, in a cosmetically acceptable medium, at least an effective amount of cinnamic acid and at least one other product which stimulates collagen synthesis.

18. Composition according to the preceding claim, **characterized in that** the product which stimulates collagen synthesis is selected from plant hormones or vitamin C and its derivatives.

19. Cosmetic composition comprising, in a cosmetically acceptable medium, at least an effective amount of cinnamic acid and at least one compound which stimulates lipid synthesis.

20. Composition according to the preceding claim, **characterized in that** the compound which stimulates lipid synthesis is a plant hormone.

## Patentansprüche

1. Verwendung einer wirksamen Menge von Zimtsäure in einer kosmetischen Zusammensetzung als Mittel, das dafür vorgesehen ist, die Synthese von Collagen zu stimulieren.

2. Verwendung nach Anspruch 1, **dadurch gekennzeichnet, dass** die Zimtsäure dazu dient, die Auswirkungen der Menopause auf das Collagen zu bekämpfen.

3. Verwendung nach Anspruch 1, **dadurch gekennzeichnet, dass** die Zimtsäure dazu dient, die Anzeichen der Hautalterung zu mildern.

4. Verwendung nach Anspruch 1, **dadurch gekennzeichnet, dass** die Zimtsäure dazu dient, die Straffung der Haut und/oder der Schleimhäute zu stimulieren.

5. Verwendung nach Anspruch 1, **dadurch gekennzeichnet, dass** die Zimtsäure dazu dient, die Glättung der Haut zu fördern und/oder die Haut zarter zu machen.

6. Verwendung nach einem der vorhergehenden Ansprüche, **dadurch gekennzeichnet, dass** die Zusammensetzung topisch auf die Haut und/ oder die Schleimhäute angewendet wird.

7. Verwendung nach einem der vorhergehenden Ansprüche, **dadurch gekennzeichnet, dass** die Zimtsäure in einer Menge von 10⁻⁶ bis 10 % des Gesamtgewichts der Zusammensetzung enthalten ist.

8. Verwendung nach einem der vorhergehenden Ansprüche, **dadurch gekennzeichnet, dass** die Zimtsäure in einer Menge von 10⁻³ bis 5 % des Gesamtgewichts der Zusammensetzung enthalten ist.

9. Verwendung nach einem der vorhergehenden Ansprüche, **dadurch gekennzeichnet, dass** die Zimtsäure mit einer weiteren Substanz kombiniert wird, die die Synthese des Collagen stimuliert.

10. Verwendung nach dem vorhergehenden Anspruch, **dadurch gekennzeichnet, dass** die weitere Substanz, die die Synthese des Collagen stimuliert, unter den pflanzlichen Hormonen oder Vitamin C oder seinen Derivaten ausgewählt wird.

11. Verwendung nach dem vorhergehenden Anspruch, **dadurch gekennzeichnet, dass** das pflanzliche Hormon ein Auxin ist.

12. Verwendung nach dem vorhergehenden Anspruch, **dadurch gekennzeichnet, dass** das Auxin unter 3-Indoylessigsäure (IAA), 4-Chlor-3-indoylessigsäure (4-C1-IAA), Phenylessigsäure (PAA), 3-Indolylbuttersäure (IBA), 2,4-Dichlorphenoxyessigsäure (2,4-D), α-Naphthalinessigsäure (α-NAA), β-Naphthoxyessigsäure, Indolylethanol, Indolylacetaldehyd und Indolylacetonitril ausgewählt wird.

13. Verwendung nach dem vorhergehenden Anspruch, **dadurch gekennzeichnet, dass** es sich bei dem Auxin um β-Napthoxyessigsäure handelt.

14. Verwendung nach einem der vorhergehenden Ansprüche, **dadurch gekennzeichnet, dass** die Zimtsäure mit einer weiteren Substanz kombiniert wird, die die Synthese von Lipiden stimuliert.

15. Verwendung nach dem vorhergehenden Anspruch, **dadurch gekennzeichnet, dass** die Substanz, die die Synthese von Lipiden stimuliert, unter den in den Ansprüchen 11 bis 13 definierten pflanzlichen Hormonen ausgewählt ist.

16. Verwendung nach einem der Ansprüche 9 bis 15, **dadurch gekennzeichnet, dass** die weitere Substanz, die die Synthese von Collagen und/ oder von Lipiden stimuliert, in einer Menge enthalten ist, die im Bereich von 10⁻⁶ bis 10 % und vorzugsweise 10⁻³ bis 5 % des Gesamtgewichts der Zusammensetzung liegt.

17. Kosmetische Zusammensetzung, die in einem kosmetisch akzeptablen Medium mindestens eine wirksame Menge von Zimtsäure in Kombination mit mindestens einem weiteren Produkt enthält, das die Collagen-Synthese stimuliert.

18. Zusammensetzung nach dem vorhergehenden Anspruch, **dadurch gekennzeichnet, dass** die Substanz, die die Synthese des Collagen stimuliert, unter den pflanzlichen Hormonen oder Vitamin C oder seinen Derivaten ausgewählt wird.

19. Kosmetische Zusammensetzung, die in einem kosmetisch akzeptablen Medium zumindest eine wirksame Menge von Zimtsäure in Kombination mit mindestens einem weiteren Produkt enthält, das die Synthese von Lipiden stimuliert.

20. Zusammensetzung nach dem vorhergehenden Anspruch, **dadurch gekennzeichnet, dass** die Substanz, die die Synthese von Lipiden stimuliert, unter den pflanzlichen Hormonen ausgewählt wird.
